# EUROPEAN PATENT APPLICATION

(11) **EP 4 005 399 A1**
(43) Date of publication of application: **01.06.2022**
(21) Application number: 20306459.7
(22) Date of filing: 27.11.2020
(51) Int. Cl.: A23L 5/42, A23P 20/10, A61K 9/28, C09D 7/61

(54) **EDIBLE COATING COMPOSITIONS COMPRISING PRECIPITATED CALCIUM CARBONATE**

(71) Applicant: ImerTech SAS, 75015 Paris (FR)
(72) Inventor: Pagis, Laure, 31500 Toulouse (FR)
(74) Representative: Haseltine Lake Kempner LLP

(57) **Abstract**

The present disclosure relates to an edible coating composition comprising precipitated calcium carbonate particulate material, wherein the BET surface area of the precipitated calcium carbonate particulate material is about 15 m²/g or less, a suspension comprising the edible coating composition and at least one solvent, an article which is at least partially coated with the edible coating composition or the suspension, and a process for producing an article, wherein the article is at least partially coated with the suspension. The present disclosure is furthermore directed to the use of the precipitated calcium carbonate particulate material (i) for at least partially replacing TiO₂ in edible coatings of nutraceutical products, pharmaceutical product, and/or food products and (ii) in an edible coating composition for improving the opacity of coatings and/or reducing crack formation of coatings.

## Description

### FIELD OF THE INVENTION

The present invention is directed to an edible coating composition comprising precipitated calcium carbonate (PCC) particulate material, a suspension comprising the edible coating composition, an article which is at least partially coated with the edible coating composition or suspension, a process for producing an article wherein the article is at least partially coated with the suspension and uses of the precipitated calcium carbonate particulate material.

### BACKGROUND OF THE INVENTION

A variety of nutraceutical and pharmaceutical products, such as tablets, mini-tables, pellets, capsules, granules and the like, are coated with an edible coating composition. Such compositions may, for example, be applied to modify the appearance of a product, e.g., its whiteness, or to protect a product from exposure to environmental conditions, e.g., light, humidity and/or oxygen from air. Furthermore, release properties of pharmaceutical or nutraceutical products can also be modified by the coating.

Hitherto, titanium dioxide (TiO₂) pigments have been used in such edible coating compositions, particularly when used to provide whiteness and/or opacity, due to its high brightness and covering power. However, the comparably strong flavour of titanium dioxide needs to compensated for by appropriate masking agents. Sugar is, in principle, suitable therefor, but undesirable due to the sweet taste and high calories of such coatings.

Furthermore, in recent years health concerns have arisen regarding the use of TiO₂ in such applications. For example, a new study causing customer and media concerns about absorption and carcinogenicity of pigment grade TiO₂. Hence, at least the partial replacement of TiO₂ is strongly desired. In addition, it is preferable that sugar in such composition is also avoided or at least the amount of sugar used is significantly reduced. However, current solutions used for the TiO₂-replacement often do not provide the coating with the same or at least a similar whiteness and/or opacity. Furthermore, in case of coatings, *e.g.,* for pharmaceutical or nutraceutical products, crack formation of the coatings needs to be avoided.

Hence, a filler or pigment is desired which is suitable for the at least partial replacement of titanium dioxide in edible coating compositions which composition have the same or at least similar properties compared with compositions containing titanium dioxide.

### SUMMARY OF THE INVENTION

The present invention is defined in the appended claims.

In accordance with a first aspect, there is provided an edible coating composition comprising
- from about 18 wt.% to about 58 wt.% of a filmogenic polymer;
- from about 0 wt.% to about 30 wt.% of one or more additives;
- from about 42 wt.% to about 82 wt.% of precipitated calcium carbonate (PCC) particulate material,
   each based on the total weight of solids in the edible coating composition, wherein the BET surface area of the precipitated calcium carbonate particulate material is about 15 m²/g or less.

In accordance with a second aspect, there is provided a suspension comprising the edible coating composition according to the first aspect and at least one solvent.

In accordance with a third aspect, there is provided an article at least partially coated with the edible coating composition according to the first aspect or the suspension according to the second aspect.

In accordance with the fourth aspect there is process for producing an article, wherein the article is at least partially coated with the suspension according to the second aspect.

In accordance with a fifth aspect there is provided the use of the precipitated calcium carbonate particulate material as defined in the first aspect for at least partially replacing TiO₂ in edible coatings of nutraceutical products and/or pharmaceutical product.

In accordance with a sixth aspect there is provided the use of the precipitated calcium carbonate particulate material as defined in the first aspect in an edible coating composition for improving the opacity of coatings and/or reducing crack formation of coatings

Certain embodiments of the present invention may provide one or more of the following advantages:
- Possibility of partial or full replacement of titanium dioxide by the precipitated calcium carbonate particulate material according to the present disclosure.
- Desired opacity and/or whiteness of such compositions.
- Provision of coatings showing no or only negligible crack formation during the coating process.
- Improved taste due to the partial or complete avoidance of titanium dioxide, and, resulting therefrom, the possibility to use less or even no sugar in the compositions.

The details, examples and preferences provided in relation to any particular one or more of the stated aspects of the present invention apply equally to all aspects of the present invention. Any combination of the embodiments, examples and preferences described herein in all possible variations thereof is encompassed by the present invention unless otherwise indicated herein, or otherwise clearly contradicted by context.

It is understood that the following description concerns exemplary embodiments of the present disclosure and shall not be limiting the scope of the claims.

### BRIEF DESCRIPTION OF THE DRAWINGS

The invention will further be illustrated by reference to the following figures:

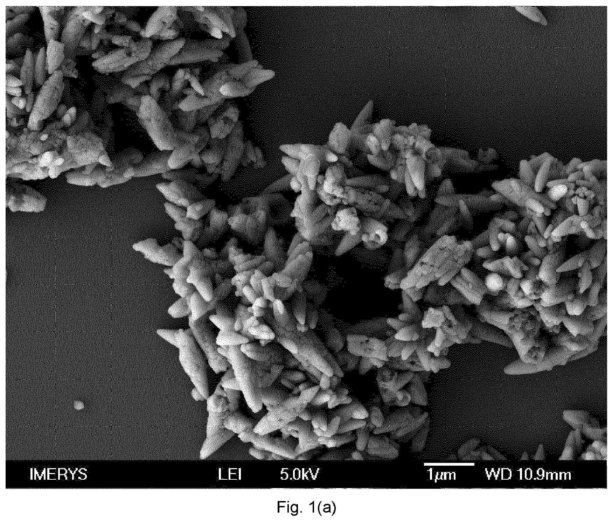

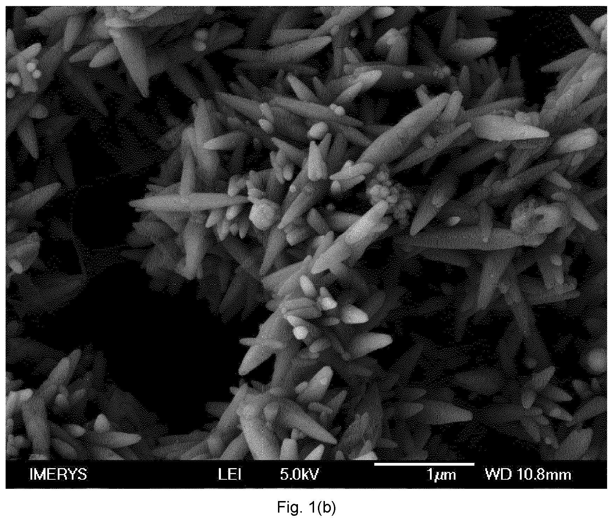

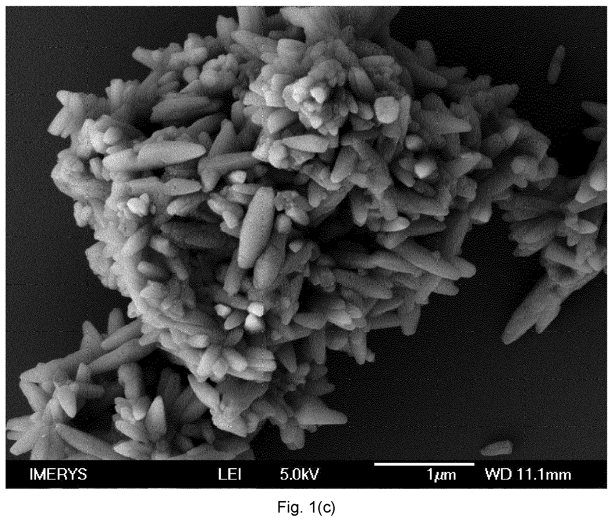

- Fig. 1(a) to (c): show microscopic images of calcium carbonate particulate materials used in the examples of the present disclosure;

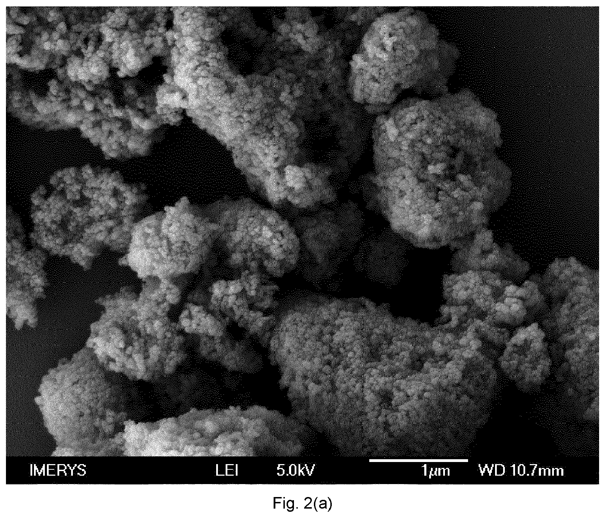

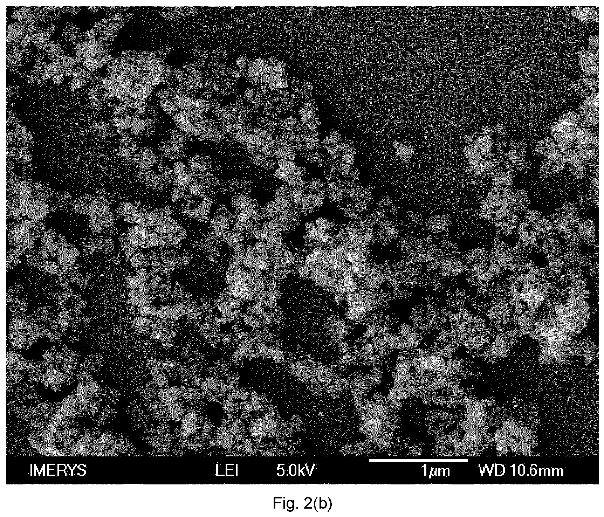

- Fig. 2(a) and (b): show microscopic images of further calcium carbonate particulate materials used in the examples of the present disclosure;
- Fig. 3(a) to (e): show the surfaces of coatings prepared in the examples.

It is understood that the following description and references to the figures concern exemplary embodiments of the present invention and shall not be limiting the scope of the claims.

### DETAILED DESCRIPTION

The present invention is based on the surprising finding that a precipitated calcium carbonate particulate material having a low BET surface area can be used in edible coating compositions, *e.g.,* as replacement for titanium dioxide.

### Method for determining the BET surface area

The surface area of the calcium carbonate particulate material is measured using the BET method according to ISO 9277, by quantitative adsorption of nitrogen on the surface of said particles so as to form a monomolecular layer completely covering said surface.

### Method for determining the oil absorption

The oil absorption is determined according to ISO 787-5 and is reported as millilitres of oil per 100 grams of calcium carbonate particulate material (ml/100 g mineral). The used oil is seed oil.

### Method for determining the particle size of the calcium carbonate particulate material

The d₅₀ particle size of the calcium carbonate particulate material is measured in a well-known manner by a Mastersizer 2000, as supplied by Malvern. The Microtrac analysis determines particle size based on the number distribution of particles using a laser light scattering technique. Such a machine provides measurements and a plot of the cumulative percentage by number of particles having a size, referred to in the art as the 'equivalent spherical diameter' (esd), less than given esd values. The median particle size d₅₀ is the value determined in this way of the particle esd at which there are 50% by number of the particles which have an equivalent spherical diameter less than that dso value.

### Method for determining the viscosity of the suspension

The viscosity is determined using a Brookfield DV-II+Pro viscometer at a temperature of 25°C using number 2 spindle at a spindle speed of 100 rpm.

### Precipitated calcium carbonate particulate material

In certain embodiments, the BET surface area of the precipitated calcium carbonate particulate material is about 15 m²/g or less, for example, less than about 15 m²/g, or about 14 m²/g or less, or about 13 m²/g or less, or about 12 m²/g or less, or about 11 m²/g or less. Usually, the BET surface area of the precipitated calcium carbonate particulate material is about 1 m²/g or more, or about 2 m²/g or more, about 3 m²/g or more. For example, the BET surface area of the precipitated calcium carbonate particulate material is from about 1 m²/g to about 15 m²/g, or from about 1 m²/g to less than about 15 m²/g or from about 1 m²/g to about 14 m²/g, or from about 2 m²/g to about 13 m²/g, or from about 2 m²/g to about 12 m²/g, or from about 3 m²/g to about 11 m²/g.

Precipitated calcium carbonate (PCC) may be produced by any of the known methods available in the art. TAPPI Monograph Series No 30, "Paper Coating Pigments", pages 34-35 describes the three main commercial processes for preparing precipitated calcium carbonate which may be used in the practice of the present disclosure. In all three processes, a calcium carbonate feed material, such as limestone, is first calcined to produce quicklime, and the quicklime is then slaked in water to yield calcium hydroxide or milk of lime. In the first process, the milk of lime is directly carbonated with carbon dioxide gas. This process has the advantage that no by-product is formed, and it is relatively easy to control the properties and purity of the calcium carbonate product. In the second process the milk of lime is contacted with soda ash to produce, by double decomposition, a precipitate of calcium carbonate and a solution of sodium hydroxide. The sodium hydroxide may be substantially completely separated from the calcium carbonate if this process is used commercially. In the third main commercial process the milk of lime is first contacted with ammonium chloride to give a calcium chloride solution and ammonia gas. The calcium chloride solution is then contacted with soda ash to produce by double decomposition precipitated calcium carbonate and a solution of sodium chloride. The crystals can be produced in a variety of different shapes and sizes, depending on the specific reaction process that is used. The three main forms of PCC crystals are aragonite, rhombohedral and scalenohedral.

In the present disclosure, the term "scalenohedral" means that the calcium carbonate particulate material has the shape of double, two-pointed pyramids which are not limited in their number of faces, *e.g.,* may be trigonal, tetragonal, *etc.*

In an exemplary embodiment, the calcium carbonate particulate material is prepared by the following process:

According to the present application, an aqueous calcium hydroxide suspension is carbonated at a starting temperature of 25°C to 70°C, or in the range of 35°C to 65°C, or in the range of 45°C to 60°C, for example by blowing a carbon dioxide containing gas. An aqueous calcium hydroxide suspension may be carbonated during at least 90 minutes, for examples at least 100 minutes. The product obtained is then dewatered in order to obtain a slurry with a high solids content, using mechanical or thermal dewatering equipment.

The precipitated calcium carbonate slurry might then be filtered, for example through a vacuum filter, or a press filter, and dried, for instance in an oven, or a flash dryer or a band dryer, or by spraying into a stream of hot air (spray drying), or by the action of radiation such as infrared radiation (epiradiator), preferably in an oven or by the action of radiation such as infrared radiation. The resulting powder might then be further milled, for instance in a pin mill with a milling intensity ranging from 1 000 rpm to 20 000 rpm. The process for making PCC results in very pure calcium carbonate crystals.

In an embodiment, the precipitated calcium carbonate particulate material comprises scalenohedral calcium carbonate particles. For example the precipitated calcium carbonate particulate material comprises about 75 wt.% or more, or about 85 wt.% or more, or about 95 wt.% or more of scalenohedral calcium carbonate particles, based on the total weight of the precipitated calcium carbonate particulate material. In an embodiment the precipitated calcium carbonate particulate material essentially consists of scalenohedral calcium carbonate particles. In this regard "essentially consists" usually denotes that the precipitated calcium carbonate particulate material comprises about 99 wt.% or more of scalenohedral calcium carbonate particles, based on the total weight of the precipitated calcium carbonate particulate material.

In an embodiment, the precipitated calcium carbonate particulate material has an oil absorption, determined according to ISO 787-5, of at least about 50 ml/100 g, for example of at least about 55 ml/100 g, or at least about 60 ml/100 g, at least about 80 ml/100 g, or at least about 95 ml/100 g. Usually, the precipitated calcium carbonate particulate material has an oil absorption, determined according to ISO 787-5, of not more than about 200 ml/100 g, for example not more than about 150 ml/100 g. For example, the precipitated calcium carbonate particulate material may have an oil absorption, determined according to ISO 787-5, of at least about 50 ml/100 g and not more than about 150 ml/100 g, or of at least about 60 ml/100 g and not more than about 110 ml/100 g.

In an embodiment, the precipitated calcium carbonate particulate material has a d₅₀ particle size, determined by laser light scattering, of about 10.0 µm or less, for example, of about 8.0 µm or less, of about 6.0 µm or less, of about 5.0 µm or less, of about 4.0 µm or less, of about 3.5 µm or less. For example, the precipitated calcium carbonate particulate material has a d₅₀ particle size, determined by laser light scattering, of about 0.5 µm or more, for example of about 1.0 µm or more. For example, the precipitated calcium carbonate particulate material has a d₅₀ particle size of from about 0.5 to about 10 µm, or from about 0.5 to about 8.0 µm, or from about 0.5 to about 6.0 µm, or from about 0.5 to about 5.0 µm, or from about 0.5 to about 4.0 µm, or from about 0.5 to about 3.5 µm, or from about 0.8 to about 3.5 µm.

### Edible coating composition

The edible coating composition according to the present disclosure comprises the precipitated calcium carbonate particulate material according to the present disclosure.

In an embodiment, the amount of precipitated calcium carbonate particulate material is from about 42 wt.% to about 79 wt.%, for example from about 52 wt.% to about 72 wt.%, or from about 57 wt.% to about 67 wt.%, based on the total weight of solids in the edible coating composition.

In the present disclosure, "filmogen" and "film-forming" are used synonymously.

In an embodiment, the edible coating composition according to the present disclosure comprises a filmogenic polymer selected from the group consisting of cellulose derived polymers, acrylate-based copolymers, methacrylate-based copolymers, homopolymers and copolymers of vinyl alcohols, homopolymers and copolymers of vinyl phenols, homopolymers and copolymers of vinyl esters, homopolymers and copolymers of ethylene oxides, homopolymer and copolymers of maleic acid, collagen, gelatin, alginates, starches, naturally occurring polysaccharides, and mixtures thereof, for example, the polymer may be selected from the group consisting of cellulose derived polymers, such as hydroxypropylcellulose, hydroxyethylcellulose, methylcellulose, ethyl cellulose, hydroxypropyl methylcellulose, cellulose acetate, cellulose acetate phthalate, carboxymethylcellulose, sodium carboxymethylcellulose, hydratecellulose, hydroxypropylmethylcellulose, hydroxypropylmethylcellulose phthalate, polymethacrylate, polyethylacrylate, compolymers of methacrylate and ethyl acrylate, methacrylic acid-ethyl acrylate copolymer, polymethylmethacrylate, copolymers of methacrylate and methylmethacrylate, polyvinylacetate and mixtures thereof. In an embodiment, the polymer is hydroxypropylmethylcellulose or polyvinylacetate; for example, the polymer is hydroxypropylmethylcellulose.

In an embodiment, the total amount of polymers is from about 21 wt.% to about 58 wt.%, for example from about 28 wt.% to about 48 wt.%, or from about 33 wt.% to about 43 wt.%, based on the total weight of solids in the edible coating composition.

In an embodiment, the edible coating composition comprises one or more additive(s) which may be selected from the group consisting of plasticizers, colourants, surfactants, detackifiers, antioxidants, flavouring agents and mixtures thereof. Such additives are usual and well-known in the art.

In an embodiment, the total amount of the one or more additive(s) is up to about 30 wt.%, for example up to about 25 wt.%, or up to about 20 wt.%, based on the total weight of solids in the edible coating composition.

In an embodiment, the edible coating composition comprises a plasticizer. For example the edible coating composition comprises a plasticizer in an amount of up to about 30 wt.%, or up to about 25 wt.%, or up to about 20 wt.%, or up to about 15 wt.%, based on the total weight of solids in the edible coating composition. If present, the plasticizer may be present in an amount of at least about 0,5 wt.%, for example of at least about 1.0 wt.%, of at least about 5.0 wt.%, of at least about 10 wt.%, or of at least about 15 wt.%, based on the total weight of solids in the edible coating composition.

Suitable plasticizers are, for example selected from the group consisting of propylene glycol, Glycerol PEG (200 - 600), Acetate ester, such as glyceryl triacetate, triethyl citrate, acetyl triethyl citrate, dibutyl subacetate, phthalate ester, acetylated monoglyceride and mixtures thereof.

As outlined above, the precipitated calcium carbonate particulate material according to the present disclosure may be used to at least partially replace TiO₂ in edible coating compositions. In an embodiment, the edible coating composition according to the present disclosure comprises less than about 10 wt.% of TiO₂, for example less than about 5 wt.%, or less than about 2.5 wt.%, or less than about 1.0 wt.% of TiO₂, based on the total weight of solids in the edible coating composition. In an embodiment, the edible coating composition according to the present disclosure is essentially free of TiO₂. In this regard, "essentially free" usually denotes that the edible coating composition according to the present disclosure comprises less than about 0.1 wt.% of TiO₂, based on the total weight of solids in the edible coating composition. In an embodiment, the edible coating composition according to the present disclosure is free of TiO₂.

In an embodiment, the edible coating composition according to the present disclosure is an edible nutraceutical coating composition or an edible pharmaceutical coating composition, for example, the edible coating composition is an edible non-sugar coating composition.

Hence, in an embodiment, the edible coating composition according to the present disclosure, for example the edible nutraceutical coating composition or an edible pharmaceutical coating composition is sugar-free.

### Suspension

Furthermore, the present disclosure is directed to a suspension comprising the edible coating composition according to the present disclosure and at least one solvent.

In one embodiment, the solvent is selected from the group consiting of water, alcohols, esters, ketones, chlorinated hydrocarbons and mixtures thereof, for example, the solvent is selected from the group consiting of water, methanol, ethanol, 2-propanol, ethyl acetate, ethyl lactate, methylene chloride and mixtures thereof, or the solvent is selected from the group consiting of water, ethanol, methylene chloride and mixtures thereof. In one embodiment, the suspension according to the present disclosure is an edible aqueous suspension. In one embodiment, the solvent essentially consists of water, for example, the solvent consists of water.

In one embodiment, the weight of the solvent is from about 60 wt.% to about 97 wt.%, for example, from about 70 wt.% to about 95 wt.%, or from about 80 wt.% to about 95 wt.%, or from about 82 wt.% to about 95 wt.%, based on the total weight of the suspension.

In one embodiment, the suspension according to the present disclosure, has a viscosity, determined using a Brookfield DV-II+Pro viscometer as disclosed herein, of up to about 150 mPa·s, for example of up to about 100 mPa·s, or of up to about 70 mPa·s. Usually, the suspension according to the present disclosure, has a viscosity, determined using a Brookfield DV-II+Pro viscometer as disclosed herein, of about 5 mPa·s or more, or of about 10 mPa·s or more, or of about 15 mPa·s or more. For example, the suspension according to the present disclosure, may have a viscosity, determined using a Brookfield DV-II+Pro viscometer as disclosed herein, of from about 5 mPa·s to about 150 mPa·s, or from about 10 mPa·s to about 100 mPa·s, or from about 15 mPa·s to about 70 mPa·s.

### Article

The present disclosure is further directed to an article which is at least partially coated with the edible coating composition or the suspension according to the present disclosure.

In an embodiment, the article is a nutraceutical product or a pharmaceutical product, for example, the article may be selected from the group consisting of, tablets, pellets, capsules, and granules.

In an embodiment, at least about 50%, for example, or at least about 60%, or at least about 70%, or at least about 80%, or at least about 90% of the surface of the article is coated with the edible coating composition or the suspension according to the present disclosure. In an embodiment, essentially the entire surface of the article, for example the entire surface of the article is coated with the edible coating composition or the suspension according to the present disclosure.

The difference between the weight of the article after coating and the weight of the article before coating is denoted weight gain.

In an embodiment, the weight gain is from about 0.5 wt.% to about 7.0 wt.%, or from 0.5 wt.% to about 6.0, or from 0.5 wt.% to about 5.0, for example from about 0.8 wt.% to 4.5 wt.%, or from about 1.1 wt.% to about 4.0 wt.%, based on the total weight of the article prior to coating.

In an embodiment, the article comprises less than about 0.16 wt.% of TiO₂, based on the total weight of the article. For example, the article comprises less than about 0.08 wt.% of TiO₂, or less than about 0.04 wt.% of TiO₂, or less than about 0.016 wt.% of TiO₂, based on the total weight of the article. In an embodiment, the article according to the present disclosure is essentially free of TiO₂. In this regard, "essentially free" usually denotes that the edible coating composition according to the present disclosure comprises less than about 0.0016 wt.% wt.% of TiO₂. In an embodiment, the article according to the present disclosure is free of TiO₂. In a variant of this embodiment, the article is a tablet.

Furthermore, embodiments and variants of the precipitated calcium carbonate material as defined in the present disclosure and the edible coating composition according to the present disclosure are also embodiment and variants of the suspension according to the present disclosure.

### Process

The present disclosure is further directed to a process for producing an article, wherein the article is at least partially coated with the suspension according to the present disclosure.

Coating processes are generally known in the art and usually depend on the article to be coated.

In one embodiment, the process comprises the following steps:
- applying the suspension according to the present disclosure to the surface of the article, e.g., by spraying or dipping, optionally, while concomitantly conveying and/or circulating the article;
- drying of the applied suspension, optionally, while concomitantly conveying and/or circulating the article.

In one embodiment, the temperature, when drying the applied suspension, is about 85°C or below, for example about 75°C or below, or about 65°C or below.

In one embodiment, a pan coater is used for coating the article, for example, in case the article is selected from the group consisting of tablets, pellets, capsules and/or granules. In a variant of this embodiment, the drum speed of the pan coater is about 30 rpm or less, for example about 24 rpm or less, or about 22 rpm or less, or about 20 rpm or less, or about 18 rpm or less. Usually, in this variant, the drum speed of the pan coater is about 3 rpm or more.

### Uses

The present disclosure is further directed to the use of the precipitated calcium carbonate particulate material as defined in the present disclosure for at least partially replacing TiO₂ in edible coatings of nutraceutical products or pharmaceutical product.

In one embodiment, the present disclosure is directed to the use of the precipitated calcium carbonate particulate material as defined in the present disclosure for essentially replacing TiO₂ in edible coatings of nutraceutical products or pharmaceutical products, for example for completely replacing TiO₂ in edible coatings of nutraceutical products or pharmaceutical products. In this regard, "essentially replacing" usually denotes that the edible coating composition according to the present disclosure comprises less than about 0.1 wt.% of TiO₂.

The present disclosure is further directed to the use of the precipitated calcium carbonate particulate material as defined in the present disclosure in an edible coating composition for improving the opacity of coatings and/or reducing crack formation of coatings.

In one embodiment, the opacity of coatings is improved and/or the crack formation of coatings is reduced compared with a comparative coating. A comparative coating is a coating not according to the present disclosure. An example of a comparative coating is a coating, wherein the precipitated calcium carbonate particulate material as defined in the present disclosure is replaced with a calcium carbonate particulate material not as defined in the present disclosure, *e.g.,* replaced with a precipitated calcium carbonate particulate material not as defined in the present disclosure. A further example of a comparative coating is a coating, wherein the precipitated calcium carbonate particulate material as defined in the present disclosure is replaced with a calcium carbonate particulate material having a BET surface area of more than about 15 m²/g, *e.g.,* replaced with a precipitated calcium carbonate particulate material having a BET surface area of more than about 15 m²/g. Yet a further example of a comparative coating is a coating, wherein the precipitated calcium carbonate particulate material as defined in the present disclosure is replaced with a calcium carbonate particulate material (i) having a BET surface area of more than about 15 m²/g and (ii) is not comprising scalenohedral calcium carbonate particles, e.g., replaced with a precipitated calcium carbonate particulate material (i) having a BET surface area of more than about 15 m²/g and (ii) is not comprising scalenohedral calcium carbonate particles. Yet a further example of a comparative coating is a coating, wherein the precipitated calcium carbonate particulate material as defined in the present disclosure is replaced with a calcium carbonate particulate material (i) having a BET surface area of more than about 15 m²/g, (ii) is not comprising scalenohedral calcium carbonate particles and (iii) having an oil absorption of less than about 50 ml/100 g, *e.g.,* replaced with a precipitated calcium carbonate particulate material (i) having a BET surface area of more than about 15 m²/g, (ii) is not comprising scalenohedral calcium carbonate particles and (iii) having an oil absorption of less than about 50 ml/100 g.

Embodiments and variants of the precipitated calcium carbonate material as defined in the present disclosure, the edible coating composition according to the present disclosure and the suspension according to the present disclosure are also embodiments and variants of the article, the process and the uses according to the present disclosure.

In some embodiments, coatings according to the present disclosure comprising the precipitated calcium carbonate particulate material as defined in the present disclosure have high opacity and/or high crack resistance. Without wishing to be bound by theory, it is currently believed that the specific BET surface area plays a crucial role in this regard.

For the avoidance of doubt, the present application is directed to subject-matter described in the following numbered paragraphs.
1. An edible coating composition comprising
   - from about 18 wt.% to about 58 wt.% of a filmogenic polymer;
   - from about 0 wt.% to about 30 wt.% of one or more additives;
   - from about 42 wt.% to about 82 wt.% of precipitated calcium carbonate (PCC) particulate material,
      each based on the total weight of solids in the edible coating composition, wherein the BET surface area of the precipitated calcium carbonate particulate material is about 15 m²/g or less.
2. The edible coating composition according to any one of the preceding numbered paragraphs, wherein the precipitated calcium carbonate particulate material comprises scalenohedral calcium carbonate particles.
3. The edible coating composition according to any one of the preceding numbered paragraphs, wherein the precipitated calcium carbonate particulate material has an oil absorption of at least about 50 ml/100 g, determined according to ISO 787-5.
4. The edible coating composition according to any one of the preceding numbered paragraphs, wherein the filmogenic polymer is selected from the group consisting of cellulose derived polymers, acrylate-based copolymers, methacrylate-based copolymers, homopolymers and copolymers of vinyl alcohols, homopolymers and copolymers of vinyl phenols, homopolymers and copolymers of vinyl esters, homopolymers and copolymers of ethylene oxides, homopolymer and copolymers of maleic acid, collagen, gelatin, alginates, starches, naturally occurring polysaccharides, and mixtures thereof.
5. The edible coating composition according to any one of the preceding numbered paragraphs, comprising less than about 10 wt.% of TiO₂, based on the total weight of solids in the edible coating composition.
6. The edible coating composition according to numbered paragraph 5, being essentially free of TiO₂.
7. The edible coating composition according to any one of the preceding numbered paragraphs, wherein the edible coating composition is an edible nutraceutical coating composition or an edible pharmaceutical coating composition.
8. The edible coating composition according to numbered paragraph 7, wherein the edible coating composition is an edible non-sugar coating composition.
9. A suspension comprising the edible coating composition according to any one of the preceding numbered paragraphs and at least one solvent.
10. The suspension according to numbered paragraph 9 being an edible aqueous suspension.
11. The suspension according to numbered paragraph 9 or numbered paragraph 10, having a viscosity of up to about 150 mPa·s, determined using a Brookfield DV-II+Pro viscometer.
12. An article at least partially coated with the edible coating composition or the suspension according to any one of the preceding numbered paragraphs.
13. The article according to numbered paragraph 12, wherein the article is a nutraceutical product or a pharmaceutical product.
14. The article according to numbered paragraph 13, wherein the article is selected from the group consisting of tablets, pellets, capsules and granules.
15. The article according to numbered paragraph 14, wherein the article is a tablet.
16. A process for producing an article, wherein the article is at least partially coated with the suspension according to any one of numbered paragraph 9 to 11.
17. Use of the precipitated calcium carbonate particulate material as defined in any one of numbered paragraphs 1 to 3 for at least partially replacing TiO₂ in edible coatings of nutraceutical products and/or pharmaceutical products.
18. Use of the precipitated calcium carbonate particulate material as defined in any one of numbered paragraphs 1 to 3 in an edible coating composition for improving the opacity of coatings and/or reducing crack formation of coatings.

### EXAMPLES

### Materials

The used calcium carbonate particulate materials and their properties are given in table 1 below.

**Table 1**

| Compound | D₅₀ particle size [µm] | Morphology | BET [m²/g] | Oil absorption [ml/100 g] |
|---|---|---|---|---|
| Compound 1 | 3.2 | "Shell family" | 29 | 82 |
| Compound 2 | 2.7 | Scalenohedral | 11 | 61 |
| Compound 3 | 1.9 | Cubic | 17 | 61 |
| Compound 4 | 3 | Scalenohedral | 9 | 105 |
| Compound 5 | 9.7 | Spherical | 70 | 115 |
| Compound 6 | 1.8 | Scalenohedral | 7 | 78 |

| | | | | |
|---|---|---|---|---|
| Compounds 1-6 are PCCs and are obtainable from Imerys. Fig. 1(a) to (c) show the scalenohedral structures of (a) Compound 2, (b) Compound 4 and (c) Compound 6. For comparison, in Fig. 2(a) and (b) the structures of (a) Compound 1 and (b) Compound 3 are shown. | | | | |

VIVAPHARM^{®} HPMC E5, a hydroxypropylmethylcellulose, obtainable from JRS Pharma, denoted "HPMC" in the following.

Yellow test tablets were obtained from Medelpharm.

### Examples:

A slurry comprising 38 wt% of HPMC and 62 wt.% the respective calcium carbonate particulate material was prepared and combined with water such that a suspension comprising 87 wt.% of water was obtained.

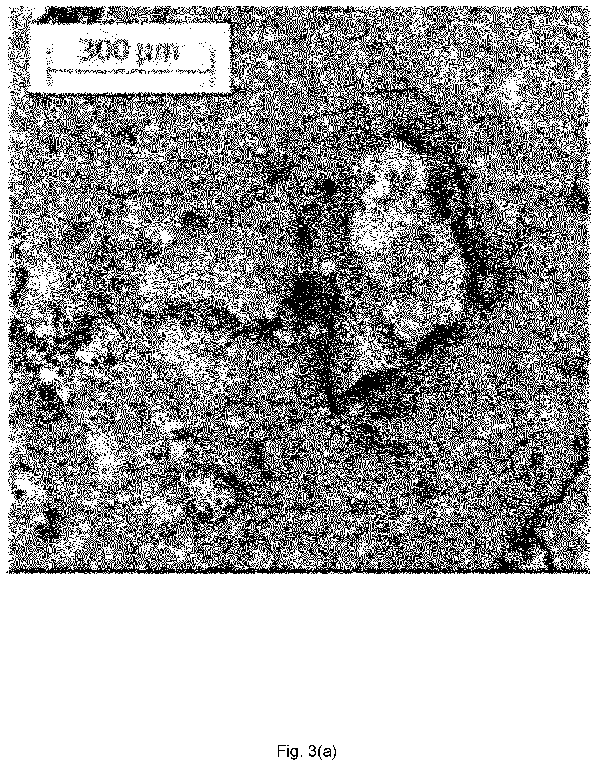

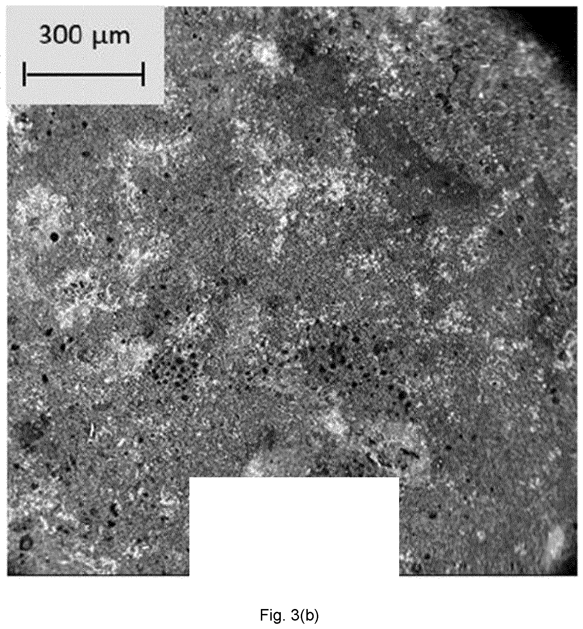

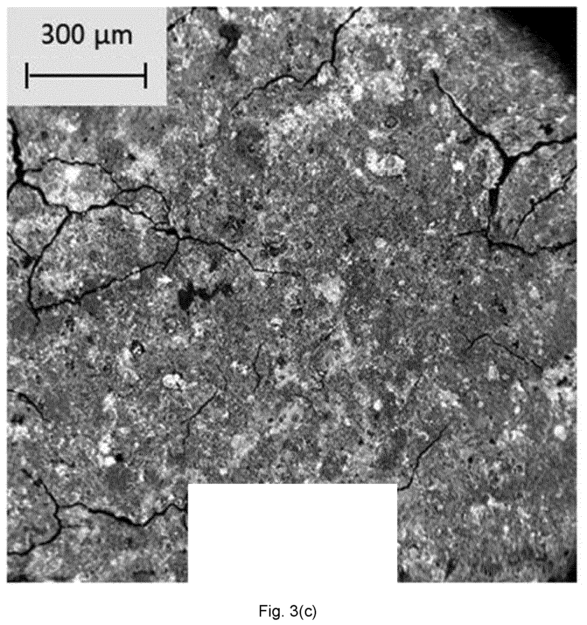

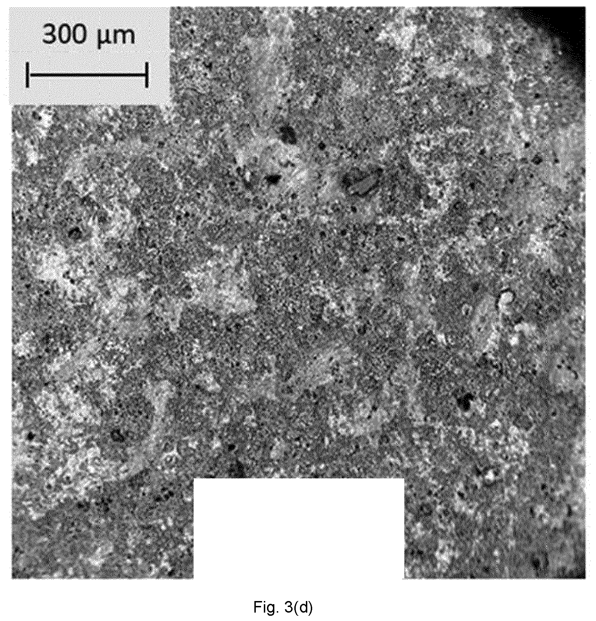

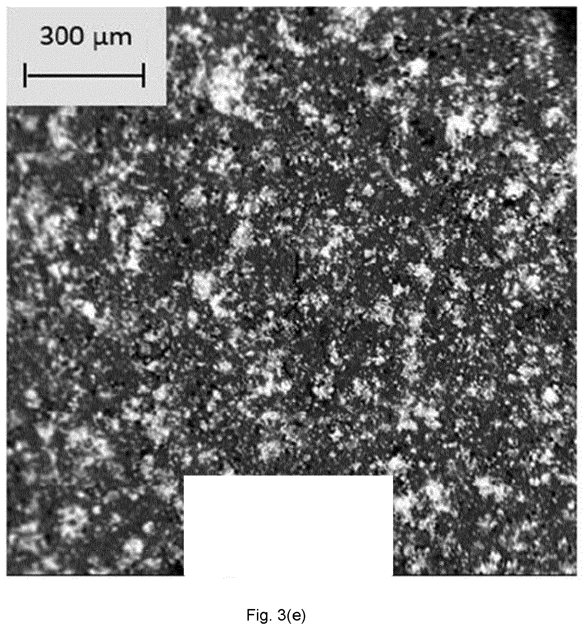

Each of these suspensions was used to coat 150 g of the test tablets in a pan coater, obtainable from ProCepT, having a pan volume of 0.35 I a nozzle orifice of 500 µm and at a drum speed of 18 rpm. The drying was performed in the pan coater at a temperature of 60°C. The weight gain is provided in table 2 below. The opacity of the resulting tablets was judged by a test panel of 5 persons by adapting the standard ISO 4121:2003 and categorized from 1 (high) to 7 (low), using a reference opacity scheme. Furthermore, using a microscope model Phenom Pro Suite and a magnification of 1:195 the coating of the tablets was visually inspected as to whether crack formation occurs. The results are provided in table 2 below. Furthermore, the crack formation is depicted in Fig. 3(a) to (e).

**Table 2**

| Grade | Opacity | Cracking | Weight gain [%] |
|---|---|---|---|
| Compound 1 (reference) | Medium (5) | Yes | n.a. |
| Compound 2 | High (4) | No | 1.58 |
| Compound 3 (reference) | Medium (5) | Yes | 1.75 |
| Compound 4 | Very High (3) | No | 3.57 |
| Compound 5 (reference) | Medium (5) | Yes | 1.55 |
| Compound 6 | High (4) | No | n.a. |

As can be seen from the examples, precipitated calcium carbonate particulate materials having a BET surface of about 15 m²/g or less provide superior opacity and avoidance of crack formation.

## Claims

1. An edible coating composition comprising
- from about 18 wt.% to about 58 wt.% of a filmogenic polymer;
- from about 0 wt.% to about 30 wt.% of one or more additives;
- from about 42 wt.% to about 82 wt.% of precipitated calcium carbonate (PCC) particulate material,
each based on the total weight of solids in the edible coating composition, wherein the BET surface area of the precipitated calcium carbonate particulate material is about 15 m²/g or less.

2. The edible coating composition according to any one of the preceding claims, wherein the precipitated calcium carbonate particulate material comprises scalenohedral calcium carbonate particles.

3. The edible coating composition according to any one of the preceding claims, wherein the precipitated calcium carbonate particulate material has an oil absorption of at least about 50 ml/100 g, determined according to ISO 787-5.

4. The edible coating composition according to any one of the preceding claims, wherein the filmogenic polymer is selected from the group consisting of cellulose derived polymers, acrylate-based copolymers, methacrylate-based copolymers, homopolymers and copolymers of vinyl alcohols, homopolymers and copolymers of vinyl phenols, homopolymers and copolymers of vinyl esters, homopolymers and copolymers of ethylene oxides, homopolymer and copolymers of maleic acid, collagen, gelatin, alginates, starches, naturally occurring polysaccharides, and mixtures thereof.

5. The edible coating composition according to any one of the preceding claims, comprising less than about 10 wt.% of TiO₂, based on the total weight of solids in the edible coating composition.

6. The edible coating composition according to claim 5, being essentially free of TiO₂.

7. The edible coating composition according to any one of the preceding claims, wherein the edible coating composition is an edible nutraceutical coating composition or an edible pharmaceutical coating composition.

8. A suspension comprising the edible coating composition according to any one of the preceding claims and at least one solvent, for example the solvent being water

9. An article at least partially coated with the edible coating composition or the suspension according to any one of the preceding claims.

10. The article according to claim 9, wherein the article is a nutraceutical product or a pharmaceutical product.

11. The article according to claim 10, wherein the article is selected from the group consisting of tablets, pellets, capsules and granules.

12. The article according to claim 11, wherein the article is a tablet.

13. A process for producing an article, wherein the article is at least partially coated with the suspension according to claim 8 .

14. Use of the precipitated calcium carbonate particulate material as defined in any one of claims 1 to 3 for at least partially replacing TiO₂ in edible coatings of nutraceutical products and/or pharmaceutical products.

15. Use of the precipitated calcium carbonate particulate material as defined in any one of claims 1 to 3 in an edible coating composition for improving the opacity of coatings and/or reducing crack formation of coatings.
